# EUROPÄISCHE PATENTSCHRIFT

(11) **EP 0 842 163 B1**
(45) Veröffentlichungstag und Bekanntmachung des Hinweises auf die Patenterteilung: **03.11.1999**
(21) Anmeldenummer: 96927571.8
(22) Anmeldetag: 22.07.1996
(51) Int. Cl.: C07D 271/06, C07D 285/08, C09B 62/503

(54) **BENZOLDERIVATE MIT EINEM HETEROCYCLISCHEN REST**
BENZENE DERIVATIVES WITH A HETEROCYCLIC GROUP
DERIVES DE BENZENE AVEC UN RESTE HETEROCYCLIQUE

(30) Priorität: 01.08.1995 DE 19528189
(43) Veröffentlichungstag der Anmeldung: 20.05.1998
(73) Patentinhaber: BASF AKTIENGESELLSCHAFT, 67056 Ludwigshafen (DE)
(72) Erfinder: HAGEN, Helmut, D-67227 Frankenthal (DE); PATSCH, Manfred, D-67157 Wachenheim (DE); WALTHER, Bernd-Peter, D-67433 Neustadt (DE); ZAMPONI, Andrea, D-69124 Heidelberg (DE)
(74) Vertreter: Karg, Jochen
(86) Internationale Anmeldenummer: EP9603224
(87) Internationale Veröffentlichungsnummer: WO9705124

(56) Entgegenhaltungen:
- EP-A- 0 037 465
- DE-A- 2 009 421
- CHEMICAL ABSTRACTS, vol. 72, no. 3, 19.Januar 1970 Columbus, Ohio, US; abstract no. 12645p, I.A. MAZUR ET AL.: "Synthesis of 2,3-dihydro and 2,3,5,6-tetrahydro derivatives of imidazo[2,1-b]thiazole" Seite 334 ; XP002016606 & KHIM.-FARM. ZH., Bd. 3, Nr. 8, 1969, Seiten 11-5,

## Beschreibung

Die vorliegende Erfindung betrifft neue Benzolderivate der Formel I 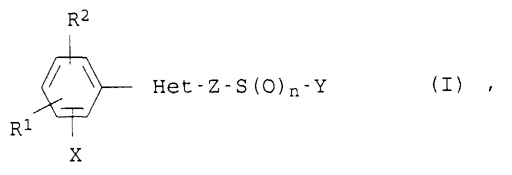 in der
- n: 0, 1 oder 2,
- X: Nitro oder Amino,
- Z: eine direkte Bindung oder C₁-C₆-Alkylen, das durch 1 oder 2 Sauerstoff- oder Schwefelatome in Etherfunktion oder 1 oder 2 nicht benachbarte Imino-, C₁-C₄-Alkylimino- oder C₁-C₄-Alkanoyliminogruppen unterbrochen sein kann,
- Y: Vinyl oder einen Rest der Formel C₂H₄-Q, worin Q für Hydroxy oder eine unter alkalischen Reaktionsbedingungen abspaltbare Gruppe steht,
- R¹ und R²: unabhängig voneinander jeweils Wasserstoff, C₁-C₄-Alkyl, C₁-C₄-Alkoxy, Halogen, Nitro, Amino, Hydroxysulfonyl oder einen Rest der Formel Z-S(O)ₙ-Y, worin n, Z und Y jeweils die obengenannte Bedeutung besitzen, und
- Het: einen 5-gliedrigen aromatischen heterocyclischen Rest, der substituiert sein kann, ausgewählt aus der Gruppe heterocyclischer Grundkörper bestehend aus 1,2,4-Oxadiazol 1,3,4-Oxadiazol, 1,2,4-Thiadiazol und 1,3,4-Thiadiazol, bedeuten.
4(5)-p-Nitrophenyl-2-(β-hydroxyethylthio)imidazol wird in Chem. Abs. 72 (1970), 12645 p erwähnt und dessen Verwendung zur Bekämpfung von Wurmkrankheiten vorgeschlagen.

EP-A-0 037 465 lehrt Azofarbstoffe mit Phenyl-1,2,4-oxidazolresten, dessen Phenylring eine Aminogruppe trägt, als Vorstufen für Farbstoffe mit mehreren Azogruppen.

Weiterhin sind der DE-A-2 009 421 Azofarbstoffe mit einem N-Phenylpyrazolrest zu entnehmen.

Aufgabe der vorliegenden Erfindung war es, neue Benzolderivate mit einem heterocyclischen Rest bereitzustellen, die weiterhin über eine Thioether-, Sulfoxid- oder Sulfonylgruppierung verfügen. Die neuen Benzolderivate sollten sich in vorteilhafter Weise zur Herstellung von Farbstoffen eignen.

Demgemäß wurden die eingangs näher bezeichneten Benzolderivate der Formel I gefunden.

Alle in der obengenannten Formel auftretenden Alkyl- und Alkylengruppen können sowohl geradkettig als auch verzweigt sein.

Reste R¹ und R² sind z.B. Methyl, Ethyl, Propyl, Isopropyl, Butyl, Isobutyl, sec-Butyl, tert-Butyl, Methoxy, Ethoxy, Propoxy, Isopropoxy, Butoxy, Isobutoxy, sec-Butoxy, tert-Butoxy, Fluor, Chlor oder Brom.

Reste Z sind z.B. CH₂, (CH₂)₂, (CH₂)₃, (CH₂)₄, (CH₂)₅, (CH₂)₆, CH(CH₃)CH₂, CH(CH₃)CH(CH₃), (CH₂)₂O(CH₂)₂, (CH₂)₃O(CH₂)₂, (CH₂)₂O(CH₂)₂O(CH₂)₂, (CH₂)₂S(CH₂)₂, (CH₂)₃S(CH₂)₂, (CH₂)₂S(CH₂)₂S(CH₂), (CH₂)₂NH(CH₂)₂, (CH₂)₃NH(CH₂)₂, (CH₂)₂NH(CH₂)₂NH(CH₂)₂,

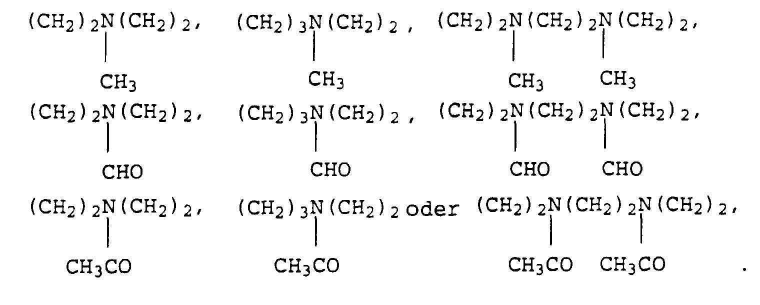

Der Rest Q steht für Hydroxy oder für eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, Phenylsulfonyloxy, C₁-C₄-Alkanoyloxy, C₁-C₄-Dialkylamino oder ein Rest der Formel 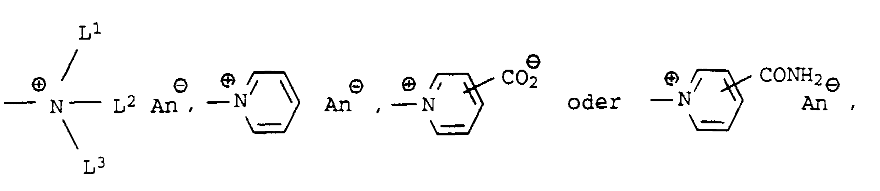 wobei L¹, L² und L³ unabhängig voneinander jeweils die Bedeutung von C₁-C₄-Alkyl oder Benzyl und An^{⊖} jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommen.

Het bedeutet den Rest eines 5-gliedrigen aromatischen heterocyclischen Ringes, ausgewählt aus der Gruppe heterocyclischer Grundkörper, die Substituenten tragen können, bestehend aus 1,2,4-Oxadiazol, 1,3,4-Oxadiazol, 1,2,4-Thiadiazol und 1,3,4-Thiadiazol.

Geeignete Substituenten für die heterocyclischen Grundkörper sind beispielsweise C₁-C₄-Alkyl, Phenyl, Halogen, Cyano, Carboxyl oder C₁-C₄-Alkoxycarbonyl.

Bevorzugt sind Benzolderivate der Formel I, in der n 0 oder 2 bedeutet.

Weiterhin bevorzugt sind Benzolderivate der Formel I, in der Z C₁-C₄-Alkylen, das durch ein Sauerstoffatom in Etherfunktion unterbrochen sein kann, bedeutet.

Weiterhin bevorzugt sind Benzolderivate der Formel I, in der Y 2-Hydroxyethyl bedeutet.

Weiterhin bevorzugt sind Benzolderivate der Formel I, in der R¹ die obengenannte Bedeutung besitzt und R² Wasserstoff bedeutet.

Weiterhin bevorzugt sind Benzolderivate der Formel I, in der Het 1,2,4-Oxadiazol-3,5-diyl bedeutet.

Besonders bevorzugt sind Benzolderivate der Formel I, in der R¹ Wasserstoff, Nitro, Amino, Hydroxysulfonyl oder einen Rest der Formel S(O)ₙC₂H₄OH worin für n 0 oder 2 steht, und R² Wasserstoff bedeutet.

Daneben werden Verbindungen der Formel I bevorzugt, in denen die Substituenten aus einer Kombination der oben aufgeführten bevorzugten Substituenten ausgewählt sind.

Von besonderem technischen Interesse sind Benzolderivate der Formel Ia oder Ib 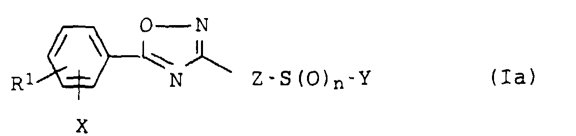 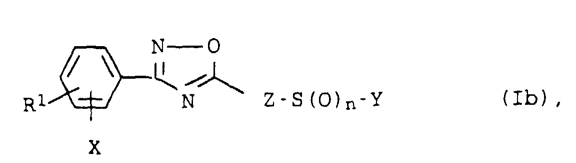 worin
- n: 0 oder 2,
- X: Nitro oder Amino,
- z: C₁-C₄-Alkylen und
- R¹: Wasserstoff, Nitro, Amino, Hydroxysulfonyl oder einen Rest der Formel S(O)ₙC₂H₄OH, worin n für 0 oder 2 steht, bedeuten und
- Y: die obengenannte Bedeutung besitzt, wobei 2-Hydroxyethyl besonders zu nennen ist.

Die neuen Benzolderivate der Formel I können auf an sich bekannten Wegen hergestellt werden. Beispielsweise kann man dabei von solchen Zwischenprodukten ausgehen, die geeignet sind, den Heterocyclus, der dem Rest Het zugrundeliegt, aufzubauen.

Dies wird im folgenden für den Fall näher erläutert, in dem Het sich von 1,2,4-Oxadiazol ableitet.

Beispielsweise kann man ein Nitril der Formel II

NC-Z-S(O)ₙ-Y (II),

in der n, Y und Z jeweils die obengenannte Bedeutung besitzen, mit Hydroxylamin umsetzen und das resultierende Amidoxim der Formel III 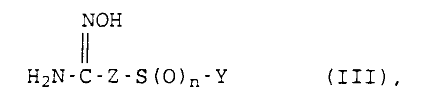 in der n, Y und Z jeweils die obengenannte Bedeutung besitzen, mit einem Isatosäureanhydrid der Formel IV 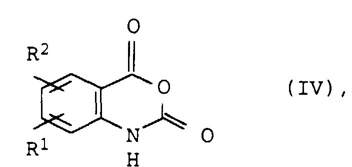 in der R¹ und R² jeweils die obengenannte Bedeutung besitzen, zur Reaktion bringen. Man gelangt so zu Benzolderivaten der Formel Ic 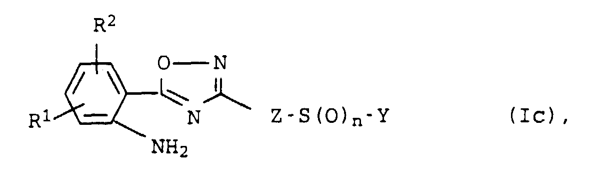 in der n, R¹, R², Y und Z jeweils die obengenannte Bedeutung besitzen.

Es ist aber auch möglich, Benzoylhalogenide der Formel VI 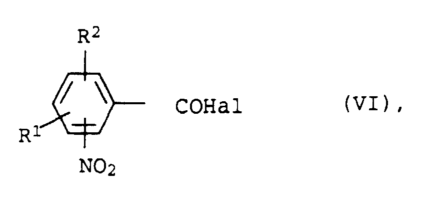 in der R¹ und R² jeweils die obengenannte Bedeutung besitzen und Hal Chlor oder Brom bedeutet, mit dem Oxim eines Halogencarbonsäureamids der Formel VII 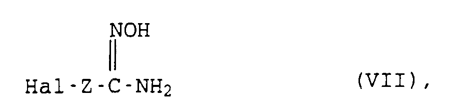 in der Hal und Z jeweils die obengenannte Bedeutung besitzen, umzusetzen und das resultierende Oxadiazol der Formel VIII 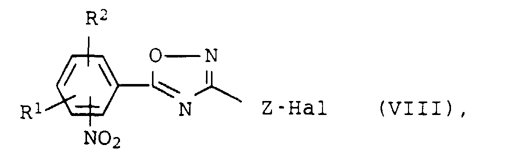 in der Hal, R¹, R² und Z jeweils die obengenannte Bedeutung besitzen, mit Mercaptoethanol reagieren zu lassen. Man erhält auf diese Weise ein Benzolderivat der Formel Id 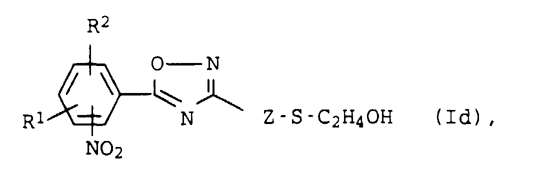 in der R¹, R² und Z jeweils die obengenannte Bedeutung besitzen. Weiterhin kann man auch ein Benzamidoxim der Formel IX 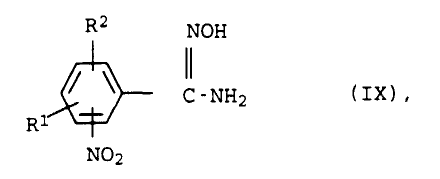 in der R¹ und R² jeweils die obengenannte Bedeutung besitzen, mit einem Halogencarbonsäurehalogenid der Formel X

Hal-Z-COHal (X),

in der Hal und Z jeweils die obengenannte Bedeutung besitzen, zur Reaktion bringen und das resultierende Oxadiazol der Formel XI 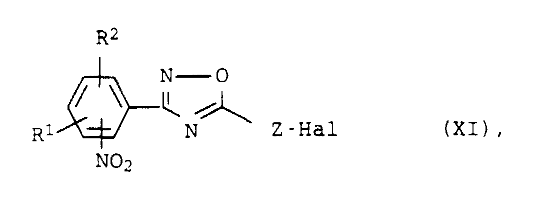 in der Hal, R¹, R² und Z jeweils die obengenannte Bedeutung besitzen, mit Mercaptoethanol umsetzen, wobei man zu einem Benzolderivat der Formel Ie 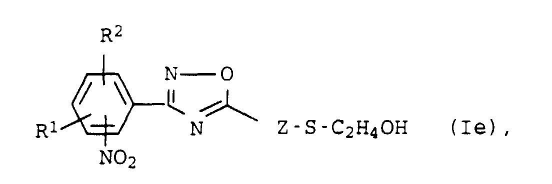 gelangt, in der R¹, R² und Z jeweils die obengenannte Bedeutung besitzen.

Gegebenenfalls kann man anschließend in den Benzolderivaten der Formel Id und Ie in beliebiger Reihenfolge und auf an sich bekanntem Wege die Nitrogruppe zur Aminogruppe reduzieren, das Schwefelatom zur Sulfoxid- oder Sulfonylgruppe oxidieren und die 2-Hydroxyethylgruppe in die Vinylgruppe oder die Gruppierung C₂H₄Q, worin Q für eine unter alkalischen Reaktionsbedingungen abspaltbare Gruppe steht, überführen.

Bei Umsetzung des Nitrils II kann man selbstverständlich von solchen Nitrilen II, die über eine Thioethergruppe (n = 0), eine Sulfoxidgruppe (n = 1) oder eine Sulfonylgruppe (n = 2) verfügen, ausgehen.

Bei den Nitrilen mit n = 0 und n = 1 kann dann die Oxidation des Schwefelatoms gegebenenfalls zu einem späteren Zeitpunkt in der Reaktionsfolge vorgenommen werden.

Dies gilt sinngemäß auch für den Rest Y im Nitril II, d.h. man kann von Edukten ausgehen, die einen Vinyl- oder 2-Hydroxyethylrest oder den Rest C₂H₄Q aufweisen, worin Q für eine unter alkalischen Reaktionsbedingungen abspaltbare Gruppe steht.

Die folgenden Beispiele sollen die Erfindung näher erläutern.

### Beispiel 1

a. 3-[(2-Hydroxyethylthio)methyl]-5-(3'-nitrophenyl)-1,2,4-oxadiazol 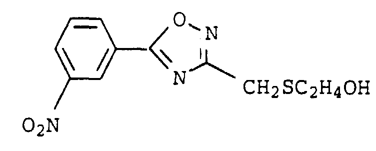
   Ein Gemisch aus 132 g (0,55 mmol) 3-Chlormethyl-5-(3'-nitrophenyl)-1,2,4-oxadiazol (zugänglich durch Umsetzung von 3-Nitrobenzoylchlorid mit Chloracetamidoxim), 39,0 ml (0,56 mol) Mercaptoethanol und 80 ml Triethylamin in 700 ml l-Methoxy-propan-2-ol wurde 4 h auf 100°C erhitzt und nach dem Abkühlen auf Eiswasser gegeben. Der dabei ausgefallene Feststoff wurde abgesaugt, mit Wasser gewaschen und unter vermindertem Druck bei 40°C getrocknet. Ausbeute 142 g (91,8 % d.Th.), schwach gelbes Pulver, Schmp. 80°C.
   ¹H-NMR ([D₆]DMSO): δ = 2,70 (t, 2H, SCH₂CH₂OH), 3,60 (br. t, 2H, CH₂OH), 3,99 (s, 2H, SCH₂-Oxadiazol), 4,88 (br. s, 1H, OH), 7,94 (t, 1H, 5'-H), 8,50-8,60 (m, 2H, 4'-H, 6'-H), 8,75 (s, 1H, 2'H).
b. 3-[(2-Hydroxyethylsulfonyl)methyl]-5-(3'-nitrophenyl)-1,2,4-oxadiazol 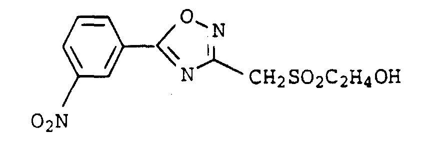
   140 g (0,50 mol) der unter a. beschriebenen Verbindung in 1 l Wasser wurden mit 40 ml Eisessig, 20 g Natriumacetat und 1 g Natriumwolframat-dihydrat versetzt und anschließend bei einer Temperatur von 70 bis 80°C innerhalb von 2 h 115 ml (1,13 mol) 30 gew.-%iges wässriges Wasserstoffperoxid zugetropft. Nach beendeter Zugabe wurde noch 2 h bei 90°C nachgerührt. Der nach dem Abkühlen ausgefallene Feststoff wurde abgesaugt, mit Wasser gewaschen und unter vermindertem Druck bei Raumtemperatur getrocknet. Ausbeute 105 g (67,0% d. Th.), schwach gelbes Pulver, Schmp. 111 bis 113°C.
   ¹H-NMR ([D₆]DMSO): δ = 3.54 (t, 2H, SO₂CH₂CH₂OH), 3,91 (q, 2H, CH₂OH), 5,00 (s, 2H, SO₂CH₂-Oxadiazol), 5,33 (t, 1H, OH), 7,97 (t, 1H, 5'-H), 8,50-8,60 (m, 2H, 4'-H, 6'-H), 8,80 (s, 1H, 2'H).
c. 5-(3'-Aminophenyl)-3-[(2-hydroxyethylsulfonyl)methyl]-1,2,4-oxadiazol 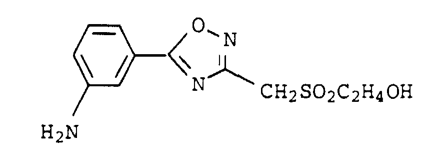
   In 110 ml (127 mmol) einer 15 gew.-%igen Lösung von Titantrichlorid in 10 gew.-%iger Salzsäure wurden 6,10 g (19,5 mmol) der unter b. beschriebenen Verbindung eingetragen und das Reaktionsgemisch 20 h bei Raumtemperatur gerührt. Vom verbliebenen Niederschlag wurde abfiltriert, die Mutterlauge mit Natronlauge alkalisch gestellt und der dabei ausgefallene Niederschlag abgesaugt und getrocknet. Anschließend wurde der Niederschlag mit 10 ml Tetrahydrofuran digeriert und vom unlöslichen Rückstand abfiltriert. Das Filtrat wurde unter vermindertem Druck zur Trockne eingeengt und der Rückstand unter vermindertem Druck bei 50°C getrocknet. Ausbeute 1,40 g (25,3 % d.Th.), ockerfarbenes Pulver, Schmp. 144 bis 145°C.
   ¹H-NMR ([D₆]DMSO): δ = 3,51 (t, 2H, SO₂CH₂CH₂OH), 3,88 (q, 2H, CH₂OH), 4,89 (s, 2H, SO₂CH₂-Oxadiazol), 5,30 (t, 1H, OH), 5,60 (br. s, 2H, NH₂), 6,86 (d, 1H, 4'-H), 7,22-7,28 (m, 2H, 5'-H, 6'-H), 7,32 (s, 1H, 2'-H).

### Beispiel 2

a. 3-[2-Hydroxyethylthio)methyl]-5-(4'-nitrophenyl)-1,2,4-oxadiazol 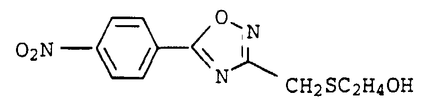
   Ein Gemisch aus 120 g (0,50 mol) 3-chlormethyl-5-(4'-nitrophenyl)-1,2,4-oxadiazol (zugänglich durch Umsetzung von 4-Nitrobenzoylchlorid mit Chloracetamidoxim), 35,0 ml (0,50 mol) Mercaptoethanol und 80 ml Triethylamin in 700 ml 1-Methoxy-propan-2-ol wurde 4 h auf 100°C erhitzt und nach dem Abkühlen auf Eiswasser gegeben. Der dabei ausgefallene Feststoff wurde abgesaugt, mit Wasser gewaschen und unter vermindertem Druck bei 50°C getrocknet. Ausbeute 131 g (93,1 % d. Th.), schwach gelbe Kristalle.
   ¹H-NMR ([D₆]DMSO): δ = 2,70 (t, 2H, SCH₂CH₂OH), 3,59 (br. t, 2H, CH₂OH) 3,98 (s, 2H, SCH₂-Oxadiazol), 4,88 (br. s, 1H, OH), 8,34 (d, 2H, 2'-H, 6'-H), 8,44 (d, 2H, 3'-H, 5'-H).
b. 3-[(2-Hydroxyethylsulfonyl)methyl]-5-(4'-nitrophenyl)-1,2,4-oxadiazol 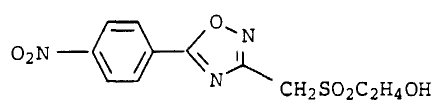
   127 g (0,45 mol) der unter a. beschriebenen Verbindung in 1 l Wasser wurden mit 40 ml Eisessig, 20 g Natriumacetat und 1 g Natriumwolframat-dihydrat versetzt und anschließend bei einer Temperatur von 70 bis 80°C innerhalb von 2 h 102 ml (1,00 mol) 30 gew.-%iges wässriges Wasserstoffperoxid zugetropft. Nach beendeter Zugabe wurde noch 2 h bei 90°C nachgerührt. Der nach dem Abkühlen ausgefallene Feststoff wurde abgesaugt, mit Wasser gewaschen und unter vermindertem Druck bei Raumtemperatur getrocknet. Ausbeute 98,2 g (69,7 % d.Th.), schwach gelbe Kristalle, Schmp. 145°C.
   ¹H-NMR ([D₆]DMSO): δ = 3,53 (t, 2H, SO₂CH₂CH₂OH), 3,90 (q, 2H, CH₂OH), 4,98 (s, 2H, SO₂CH₂-Oxadiazol), 5,32 (t, 1H, OH), 8,37 (d, 2H, 2'-H, 6'H), 8,47 (d, 2H, 3'-H, 5'-H).
c. 5-(4'-Aminophenyl)-3-[(2-hydroxyethylsulfonyl)methyl]-1,2,4-oxadiazol 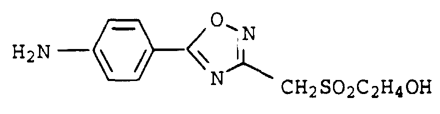
   95 g (0,30 mol) der unter b. beschriebenen Verbindung wurden in 1 l Tetrahydrofuran gelöst und unter Zusatz von 5 g 10 gew.-%igem Palladium auf Kohle unter Normaldruck bis zum Stillstand der Wasserstoffaufnahme hydriert. Es wurde vom Katalysator abfiltriert, das Lösungsmittel unter vermindertem Druck abdestilliert und der Rückstand unter vermindertem Druck bei Raumtemperatur getrocknet. Ausbeute 80,0 g (95.1 % d.Th.), gelbes Pulver, Schmp. 150 bis 152°C.
   ¹H-NMR ([D₆]DMSO): δ = 3,53 (t, 2H, SO₂CH₂CH₂OH), 3,90 (br. t, 2H, CH₂OH) 4,84 (s, 2H, SO₂CH₂-Oxadiazol), 5,30 (br. s, 1H, OH), 6,95 (d, 2H, 3'-H, 5'-H), 7,91 (d, 2H, 2'-H, 6'-H).

### Beispiel 3

a. 3-(2-Hydroxyethylsulfonyl)propionsäurenitril

   NC-C₂H₄SO₂C₂H₄OH

   328 g (2,50 mol) 3-[(2-Hydroxyethyl)thio]propionsäurenitril in 500 ml Wasser wurden mit 10 ml Eisessig, 2 g Natriumacetat und 2 g Natriumwolframat-dihydrat versetzt und anschließend bei einer Temperatur von 60 bis 70°C innerhalb von 4 h 520 ml (5,09 mol) 30 gew.-%iges wässriges Wasserstoffperoxid zugetropft. Um den angegebenen Temperaturbereich zu halten, mußte zeitweise mit Eiswasser gekühlt werden. Nach dem Abkühlen wurde überschüssiges Wasserstoffperoxid mit Natriumdlthionit vernichtet und das Lösungsmittel unter vermindertem Druck abdestilliert. Der ölige Rückstand wurde mit 200 ml Aceton digeriert und vom Niederschlag abfiltriert. Das Filtrat wurde unter vermindertem Druck zur Trockne eingeengt. Es fielen 405 g eines zähflüssigen Öls mit einem Reingehalt an 3-(2-Hydroxyethylsulfonyl)propionsäurenitril von 86,4% an (85,8% d. Th.).
   ¹H-NMR ([D₆]DMSO): δ = 2,98 (t, 2H, CH₂CN), 3,32 (t, 2H, SO₂CH₂CH₂OH), 3,49 (t, 2H, SO₂CH₂CH₂CN), 3,78 (br. t, 2H, CH₂OH), 5,22 (br. s, 1H, OH).
   (siehe auch EP-A 639 562).
b. 3-(2-Hydroxyethylsulfonyl)propionamidoxim 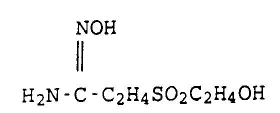
   Zu einer Lösung von 163 g (1,00 mol) der unter a. beschriebenen Verbindung und 69,5 g (1,00 mol) Hydroxylammoniumchlorid in 1 l Methanol wurden 190 ml (1,00 mol) einer 30 gew.-%igen Lösung von Natriummethanolat in Methanol getropft. Das Reaktionsgemisch wurde 6 h unter Rückfluß zum Sieden erhitzt. Nach dem Abkühlen wurde der Niederschlag abgesaugt und unter vermindertem Druck bei 50°C getrocknet. Es wurden 151 g eines hellbraunen mikrokristallinen Pulvers mit einem Gehalt an 3-(2-Hydroxyethylsulfonyl)propionamidoxim von 71,4 % erhalten (55,0 % d.Th.).
   ¹H-NMR ([D₆]DMSO): δ = 2,45 (t, 2H, CH₂C(NH₂)=NOH), 3,22-3,38 (m, 4H, CH₂SO₂CH₂), 3,80 (br. t, 2H, CH₂OH), 5,26 (br. s, 1H, CH₂OH) 5,56 (br. s, 2H, NH₂), 9,02 (br. s, 1H, N-OH).
c. 5-(2'-Aminophenyl)-3-[2-(2-hydroxyethylsulfonyl)ethyl]-1,2,4-oxadiazol 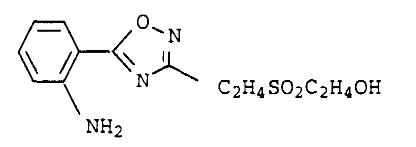
   In eine auf 50°C erwärmte Lösung von 98,1 g (0,50 mol) der unter b. beschriebenen Verbindung in 1 l 1-Methoxypropan-2-ol wurden 81,6 g (0,50 mol) Isatosäureanhydrid langsam eingetragen und das Reaktionsgemisch nach Beendigung der Gasentwicklung 6 h unter Rückfluß zum Sieden erhitzt. Das Lösungsmittel wurde anschließend unter vermindertem Druck abdestilliert, der verbliebene Rückstand mit Isopropanol überschichtet und der ausgefallene Niederschlag abgesaugt. Ausbeute 76,8 g (51,7% d. Th.), beiges Pulver, Schmp. 118 bis 119°C.
   ¹H-NMR ([D₆]DMSO): δ = 3,25 (t, 2H, CH₂-Oxadiazol), 3,35 (t, 2H, SO₂CH₂CH₂OH), 3,63 (t, 2H, SO₂CH₂CH₂-Oxadiazol), 3,77 (q. 2H, CH₂OH), 5,19 (t, 1H, OH), 6,65 (t, 1H, 5'-H), 6,87 (br. s, 2H, NH₂), 6,90 (d, 1H, 3'-H), 7,30 (t, 1H, 4'-H), 7,76 (d, 1H, 6'-H).
   MS (EI): m/z (M⁺) = 297.

### Beispiel 4

a. 5-(Chlormethyl)-3-(4'-chlor-3'-nitrophenyl)-1,2,4-oxadiazol 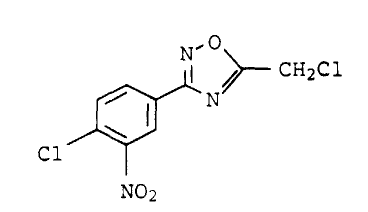
Zu einer Lösung von 323 g (1,50 mol) 4-Chlor-3-nitrobenzamidoxim und 130 ml (1,61 mol) Pyridin in 2 l Dioxan wurden 127 ml (1,60 mol) Chloracetylchlorid getropft. Das Reaktionsgemisch wurde anschließend 4 h zum Rückfluß erhitzt und nach dem Abkühlen in 3 l Eiswasser eingerührt. Der Niederschlag wurde abgesaugt, mit Wasser gewaschen und unter vermindertem Druck bei Raumtemperatur getrocknet. Ausbeute 300 g (73,0% d. Th.), schwach gelbes Pulver, Schmp. 55 bis 56°C.
¹H-NMR ([D₆]DMSO): δ = 5,23 (s, 2H, CH₂Cl), 8,00 (d, 1H, 5'-H), 8,28 (d, 1H, 6'-H), 8,61 (s, 1H, 2'-H).
b. 5-[(2-Hydroxyethylthio)methyl]-3-[4'-(2-hydroxyethyl-thio)-3'-nitrophenyl]-1,2,4-oxadiazol 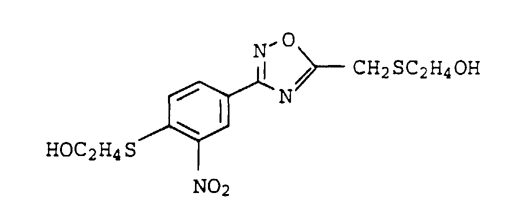
Zu einer Lösung von 164 g (0,60 mol) der unter a. beschriebenen Verbindung und 210 ml Triethylamin in 1 l 1-Methoxypropan-2-ol wurden 105 ml (1,50 mol) Mercaptoethanol getropft. Das Reaktionsgemisch wurde anschließend 6 h zum Rückfluß erhitzt und nach dem Abkühlen in 1,5 l Eiswasser eingerührt. Der Niederschlag wurde abgesaugt, mit Wasser gewaschen und unter vermindertem Druck bei Raumtemperatur getrocknet. Ausbeute 170 g (79,3% d. Th.), gelbes Pulver, Schmp. 106 bis 107°C.
¹H-NMR ([D₆] DMSO): δ = 2,77 (t, 2H, CH₂SCH₂CH₂OH), 3,25 (t, 2H, Aryl-SCH₂CH₂OH), 3,61 (q, 2H, CH₂SCH₂CH₂OH), 3,73 (q, 2H, Aryl-SCH₂CH₂OH), 4,24 (s, 2H, Oxadiazol-CH₂S), 4,91 (t, 1H, CH₂SCH₂CH₂OH), 5,15 (t, 1H, Aryl-SCH₂CH₂OH), 7,87 (d, 1H, 5'-H), 8,21 (d, 1H, 6'-H), 8,67 (s, 1H, 2'-H).
c. 5-[(2-Hydroxyethylsulfonyl)methyl]-3-[4'-(2-hydroxyethylsulfonyl)-3'-nitrophenyl]-1,2,4-oxadiazol 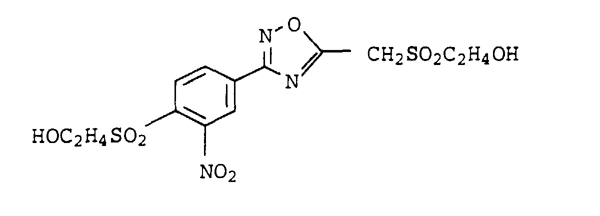
143 g (0.40 mol) der unter b. beschriebenen Verbindung in 1 l Wasser wurden mit 30 ml Eisessig, 20 g Natriumacetat und 2 g Natriumwolframat-dihydrat versetzt und bei einer Temperatur von 80°C innerhalb von 2 h 150 ml (1,47 mol) 30 gew.-%iges wässriges Wasserstoffperoxid zugetropft. Anschließend wurde zum Rückfluß erhitzt, innerhalb von 2 h weitere 150 ml (1,47 mol) 30 gew.-%iges wässriges Wasserstoffperoxid zugetropft und 3 h unter Rückfluß nachgerührt. Der nach dem Abkühlen ausgefallene Feststoff wurde abgesaugt, mit wenig kaltem Wasser gewaschen und unter vermindertem Druck bei 50°C getrocknet. Ausbeute 104 g (61,7 % d.Th.), schwach gelbe Kristalle, Schmp. 126°C.
¹H-NMR ([D₆]DMSO): δ = 3,58 (t, 2H, CH₂SO₂CH₂CH₂OH), 3,79-3,92 (m, 6H, Aryl-SO₂CH₂CH₂OH, CH₂SO₂CH₂CH₂OH, Aryl-SO₂-CH₂CH₂OH), 5,06 (br. s, 1H, Aryl-SO₂CH₂CH₂OH), 5,31 (s, 2H, Oxadiazol-CH₂SO₂), 5,37 (br. s, 1H, CH₂SO₂CH₂CH₂OH), 8,31 (d, 1H, 5'-H), 8,53 (d, 1H, 6'-H), 8,63 (s, 1H, 2'-H).
d. 3-[3'-Amino-4'-(2-hydroxyethylsulfonyl)phenyl]-5-[(2-hydroxyethylsulfonyl)methyl]-1,2,4-oxadiazol 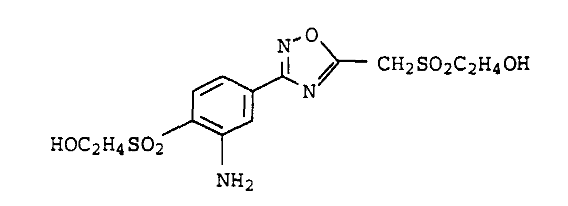
In 1,4 l (1,62 mol) einer 15 gew.-%igen Lösung von Titantrichlorid in 10 gew.-%iger Salzsäure wurden 105 g (0,25 mol) der unter c. beschriebenen Verbindung eingetragen und das Reaktionsgemisch 20 h bei Raumtemperatur gerührt. Der Niederschlag wurde abgesaugt, mit wenig Wasser gewaschen und getrocknet. Ausbeute 90,3 g als Hydrochlorid (84,4 % d.Th.), hellbraune Kristalle, Schmp. 157°C (Zers.).
Um die freie Base zu erhalten, wurde das Produkt in Wasser suspendiert und mit wäßriger Ammoniaklösung neutralisiert. Der Niederschlag wurde abgesaugt und aus Methanol umkristallisiert.

| | | | | | | |
|---|---|---|---|---|---|---|
| Analyse | ber. | C 39,9 | H 4,4 | N 10,7 | O 28,6 | S 16,4 |
| | gef. | C 39,7 | H 4,4 | N 10,7 | O 28,2 | S 16,4 |

MS (ESI): m/z (M+H⁺) = 392.
¹H-NMR ([D₆]DMSO) : δ = 3,44 (t, 2H, Aryl-SO₂CH₂CH₂OH), 3,58 (t, 2H, CH₂SO₂CH₂CH₂OH), 3,69 (q, 2H, Aryl-SO₂CH₂CH₂OH). 3,88
(q, 2H, CH₂SO₂CH₂CH₂OH), 4,89 (t, 1H, Aryl-SO₂CH₂CH₂OH), 5,26 (s, 2H, Oxadiazol-CH₂SO₂), 5,35 (t, 1H, CH₂SO₂CH₂CH₂OH), 6,39 (br. s, 2H, NH₂), 7,27 (d, 1H, 6'-H), 7,61 (s, 1H, 2'-H), 7,66 (d, 1H, 5'-H).

### Beispiel 5

a. 3-(2-Hydroxyethylthio)propionamidoxim 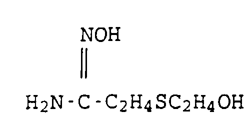
   Zu einer Lösung von 131 g (1,00 mol) 3-(2-Hydroxyethylthio)propionsäurenitril und 69,5 g (1,00 mol) Hydroxylammoniumchlorid in 1 l Methanol wurden 190 ml (1,00 mol) einer 30 gew.-%igen Lösung von Natriummethanolat in Methanol getropft. Das Reaktionsgemisch wurde 6 h zum Rückfluß erhitzt. Nach dem Abkühlen wurde vom Niederschlag abgesaugt und das Filtrat unter vermindertem Druck zur Trockne eingeengt. Es fielen 152 g eines zähflüssigen Öls mit einem Reingehalt an 3-(2-Hydroxyethylthio)propionamidoxim von 82,4 % an (76,3 % d. Th.).
   ¹H-NMR ([D₆]DMSO): δ = 2,20 (t, 2H, CH₂C(NH₂)=NOH), 2,57 (t, 2H, SO₂CH₂), 2,68 (t, 2H, SO₂CH₂), 3,52 (br. t, 2H, CH₂OH), 4,79 (br. s, 1H, CH₂OH), 5,40 (br. s, 2H, NH₂), 8,81 (br. s, 1H, N-OH).
b. 5-(2'-Aminophenyl)-3-[2-(2-hydroxyethylthio)ethyl]-1,2,4-oxadiazol 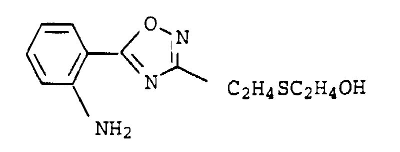
   In eine auf 50°C erwärmte Lösung von 82,1 g (0,50 mol) der unter a. beschriebenen Verbindung in 1 l 1-Methoxypropan-2-ol wurden 81,6 g (0,50 mol) Isatosäureanhydrid langsam eingetragen und das Reaktionsgemisch nach Beendigung der Gasentwicklung 6 h zum Rückfluß erhitzt. Das Lösungsmittel wurde anschließend unter vermindertem Druck abdestilliert und der Rückstand mit Isopropanol überschichtet. Der Niederschlag wurde abgesaugt und getrocknet. Ausbeute 80,9 g (61,0 % d.Th.) beiges Pulver.
   ¹H-NMR ([D₆]DMSO): δ = 2,95 (t, 2H, CH₂-Oxadiazol), 2,70 (t, 2H, SO₂CH₂CH₂OH), 3,01 (t, 2H, SO₂CH₂CH₂-Oxadiazol, 3,47 (q, 2H, CH₂OH), 4,74 (t, 1H, OH), 6,62 (t, 1H, 5'-H), 6,85 (br.s., 2H, NH₂), 6,92 (d, 1H, 3'-H), 7,22 (t, 1H, 4'-H), 7,71 (d, 1H, 6'-H).
c. 5-(2'-Aminophenyl)-3-[2-(2-hydroxyethylsulfonyl)ethyl]-1,2,4-oxadiazol 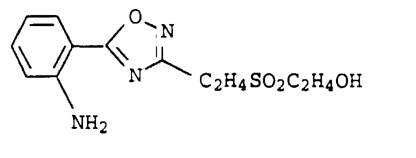
   79,7 g (0,30 mol) der unter b. beschriebenen Verbindung in 150 ml 30 gew.-%iger Schwefelsäure wurden mit 0,5 g Natriumwolframat-dihydrat versetzt. Anschließend wurde bei einer Temperatur von 60 bis 70°C innerhalb von 2 h 65 ml (0,64 mol) 30 gew.-%iges wässriges Wasserstoffperoxid zugetropft. Nach dem Abkühlen wurde das Reaktionsgemisch auf 500 g Eis gegeben und mit verdünnter Natronlauge neutral gestellt. Der Niederschlag wurde abgesaugt und dreimal mit je 80 ml Methanol gewaschen. Ausbeute 72,8 g (81,6 % d. Th.), beiges Pulver, Schmp. 118 bis 110°C.
   ¹H-NMR ([D₆]DMSO): δ = 3,25 (t, 2H, CH₂-Oxadiazol), 3,35 (t, 2H, SO₂CH₂CH₂OH), 3,63 (t, 2H, SO₂CH₂CH₂-Oxadiazol), 3,77 (q, 2H, CH₂OH), 5,19 (t, 1H, OH), 6,65 (t, 1H, 5'-H), 6,87 (br. s, 2H, NH₂), 6,90 (d, 1H, 3'-H), 7,30 (t, 1H, 4'-H), 7,76 (d, 1H, 6'-H).
   MS (EI): m/z (M⁺⁾ = 297.

### Beispiel 6

a. 3-(4'-chlor-3'-nitrophenyl)-5-vinyl-1,2,4-oxadiazol 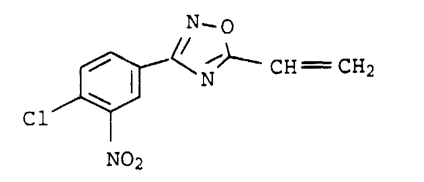
   Zu einer Lösung von 151 g (0,70 mol) 4-Chlor-3-nitrobenzamidoxim und 60 ml (0,74 mol) Pyridin in 1,5 l Dioxan wurden 58 ml (0,71 mol) Acrylsäurechlorid zugetropft und das Reaktionsgemisch anschließend 12 h zum Rücken erhitzt. Nach dem Abkühlen wurde die Dioxanphase von unlöslichen Bestandteilen abdekantiert, das Lösungsmittel im Vakuum abdestilliert und der Rückstand in 1,5 l Eiswasser eingerührt. Der ausgefallene Feststoff wurde abgesaugt, mit Wasser gewaschen, aus Methanol umkristallisiert und im Vakuum bei Raumtemperatur getrocknet. Ausbeute 130 g (74,0 % d.Th.), schwach gelbes Pulver, Schmp. 86-88°C.
   ¹H-NMR ([D₆]DMSO): d = 6,24 (d, 1H, cis-CH=CH₂), 6,66 (d, 1H, trans-CH=CH₂), 7,02 (dd, 1H, CH=CH₂), 8,03 (d, 1H, 5'-H), 8,32 (d, 1H, 6'-H), 8,64 (s, 1H, 2'-H).
b. 5-[(2-Hydroxy-ethylmercapto)-ethyl]-3-[4'-(2-hydroxy-ethylmercapto)-3'-nitrophenyl]-1,2,4-oxadiazol 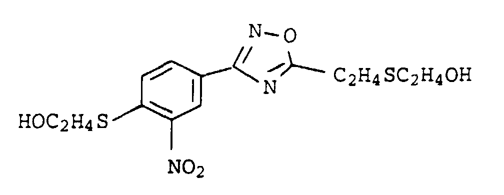
   Zu einer Lösung von 126 g (0,50 mol) des unter a. hergestellten 3-(4'-Chlor-3'-nitrophenyl)-5-vinyl-1,2,4-oxadiazol und 5 ml (0,05 mol) Piperidin in 800 ml 1-Methoxy-propan-2-ol wurden 35 ml (0,50 mol) Mercaptoethanol getropft und das Reaktionsgemisch 6 h bei 50°C gerührt. Anschließend wurden weitere 35 ml (0,50 mol) Mercaptoethanol sowie 70 ml (0,50 mol) Triethylamin zugetropft und das Reaktionsgemisch 10 h zum Rückfluß erhitzt. Nach dem Abkühlen wurde das Lösungsmittel im Vakuum abdestilliert und der Rückstand in 1 l Eiswasser eingerührt. Der ausgefallene Niederschlag wurde abgesaugt, mit Wasser und Methanol gewaschen und im Vakuum bei Raumtemperatur getrocknet. Ausbeute 153 g (82,4 % d.Th.), gelbes Pulver, Schmp. 85-88°C.
   ¹H-NMR ([D₆]DMSO): d = 2,67 (t, 2H, CH₂CH₂SCH₂CH₂OH), 3,06 (t, 2H, CH₂CH₂SCH₂CH₂OH), 3,24 (t, 2H, Aryl-SCH₂CH₂OH), 3,35 (t, 2H, CH₂CH₂SCH₂CH₂OH), 3,59 (br. t, 2H, CH₂CH₂SCH₂CH₂OH), 3,74 (br. t, 2H, Aryl-SCH₂CH₂OH), 4,87 (br. s, 1H, CH₂SCH₂CH₂OH), 5,16 (br. s, 1H, Aryl-SCH₂CH₂OH), 7,84 (d, 1H, 5'-H), 8,18 (d, 1H, 6'-H), 8,64 (s, 1H, 2'-H).
c. 5-[(2-Hydroxy-ethylsulfonyl)-ethyl]-3-[4'-(2-hydroxy-ethylsulfonyl)-3'-nitrophenyl]-1,2,4-oxadiazol 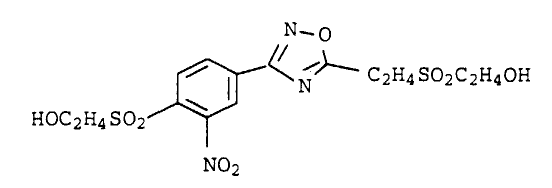
   149 g (0,40 mol) des unter b. hergestellten 5-[(2-Hydroxyethylmercapto)-ethyl]-3-[4'-(2-hydroxy-ethylmercapto)-3'-nitrophenyl]-1,2,4-oxadiazol in 1 l Wasser wurden mit 50 ml Eisessig, 20 g Natriumacetat und 2 g Natriumwolframat-dihydrat versetzt. Bei einer Temperatur von 80°C wurde innerhalb von 2 h 130 ml (1,27 mol) einer 30 gew.-%igen wäßrigen Wasserstoffperoxidlösung zugetropft. Anschließend wurde zum Rückfluß erhitzt, innerhalb von 2 h weitere 130 ml (1,27 mol) einer 30 gew.-%igen wäßrigen Wasserstoffperoxidlösung zugetropft und 3 h zum Rückfluß erhitzt. Der nach dem Abkühlen ausgefallene Feststoff wurde abgesaugt, mit Wasser gewaschen, aus n-Butanol umkristallisiert und im Vakuum bei 50°C getrocknet. Ausbeute 126 g (72,3 % d.Th.), schwach gelbe Kristalle, Schmp. 140-142°C.
   ¹H-NMR ([D₆]DMSO): δ = 3,38 (t, 2H, CH₂CH₂SO₂CH₂CH₂OH), 3,57 (t, 2H, CH₂CH₂SO₂CH₂CH₂OH), 3,77 (t, 2H, CH₂CH₂SO₂CH₂CH₂OH), 3,81-3,87 (m, 6H, Aryl-SO₂CH₂CH₂OH, CH₂CH₂SO₂CH₂CH₂OH, Aryl-SO₂CH₂CH₂OH), 5,05 (br.s, 1H, Aryl-SO₂CH₂CH₂OH), 5,25 (br.s, 1H, CH₂CH₂SO₂CH₂CH₂OH), 8,30 (d, 1H, 5'-H), 8,50 (d, 1H, 6'-H), 8,59 (s, 1H, 2'-H).
d. 3-[3'-Amino-4'-(2-hydroxy-ethylsulfonyl)phenyl]-5-[(hydroxyethylsulfonyl)-ethyl]-1,2,4-oxadiazol 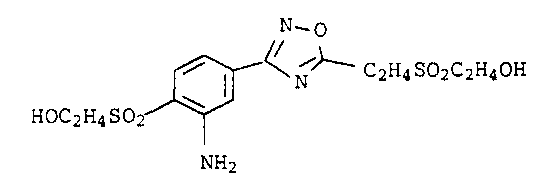
   43,5 g (0,10 mol) 5-[(2-Hydroxy-ethylsulfonyl)-ethyl]-3-[4'-(2-hydroxy-ethylsulfonyl)-3'-nitrophenyl]-1,2,4-oxadiazol (unter c. hergestellt) in 300 ml 1-Methoxypropan-2-ol wurden auf 60°C erwärmt, 800 ml Wasser zugegeben und im Verlauf von 3 h insgesamt 25 g Eisenmehl eingetragen. Dabei wurde der pH-Wert durch Zudosierung von Eisessig bei 3,5 bis 4,0 gehalten. Nach dem Abkühlen wurde das Reaktionsgemisch abfiltriert und das Lösungsmittel aus der Mutterlauge im Vakuum abdestilliert. Der dabei verbliebene Rückstand wurde mit Methanol aufgerührt, der ausgefallene Niederschlag abgesaugt, aus n-Butanol umkristallisiert und im Vakuum bei 50°C getrocknet. Ausbeute 22 g (54,3 % d.Th.), hellbraunes Pulver, Schmp. 208-210°C (Zers.).
   MS (ESI): m/z ([M-H]⁺) = 404, ([M+CI]⁺) = 440.

## Patentansprüche

1. Benzolderivate der Formel I 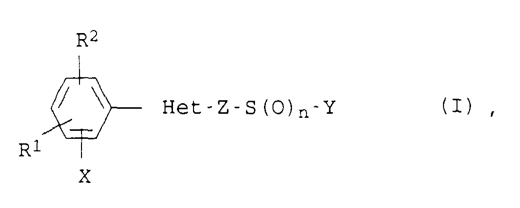 in der
n 0, 1 oder 2,
X Nitro oder Amino,
Z eine direkte Bindung oder C₁-C₆-Alkylen, das durch 1 oder 2 Sauerstoff- oder Schwefelatome in Etherfunktion oder 1 oder 2 nicht benachbarte Imino-, C₁-C₄-Alkylimino- oder C₁-C4-Alkanoyliminogruppen unterbrochen sein kann,
Y Vinyl oder einen Rest der Formel C₂H₄-Q, worin Q für Hydroxy oder eine unter alkalischen Reaktionsbedingungen abspaltbare Gruppe steht,
R¹ und R² unabhängig voneinander jeweils Wasserstoff, C₁-C₄-Alkyl, C₁-C₄-Alkoxy, Halogen, Nitro, Amino, Hydroxysulfonyl oder einen Rest der Formel Z-S(O)ₙ-Y, worin n, Z und Y jeweils' die obengenannte Bedeutung besitzen, und
Het einen 5-gliedrigen aromatischen heterocyclischen Rest, der substituiert sein kann, ausgewählt aus der Gruppe heterocyclischer Grundkörper bestehend aus 1,2,4-Oxadiazol, 1,3,4-Oxadiazol, 1,2,4-Thiadiazol und 1,3,4,Thiadiazol, bedeuten.

2. Benzolderivate nach Anspruch 1, dadurch gekennzeichnet, daß n 0 oder 2 bedeutet.

3. Benzolderivate nach Anspruch 1, dadurch gekennzeichnet, daß Z C₁-C₄-Alkylen, das durch ein Sauerstoffatom in Etherfunktion unterbrochen sein kann, bedeutet.

4. Benzolderivate nach Anspruch 1, dadurch gekennzeichnet, daß Y 2-Hydroxyethyl bedeutet.

5. Benzolderivate nach Anspruch 1, dadurch gekennzeichnet, daß R¹ die in Anspruch 1 genannte Bedeutung besitzt und R² Wasserstoff bedeutet.

6. Benzolderivate nach Anspruch 1, dadurch gekennzeichnet, daß Het 1,2,4-Oxadiazol-3,5-diyl bedeutet.

7. Benzolderivate nach Anspruch 1, dadurch gekennzeichnet, daß R¹ Wasserstoff, Nitro, Amino, Hydroxysulfonyl oder einen Rest der Formel S(O)ₙC₂H₄OH, worin für n 0 oder 2 steht, und R² Wasserstoff bedeuten.

8. Benzolderivate nach Anspruch 1, die der Formel Ia oder Ib 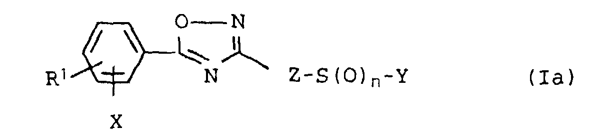 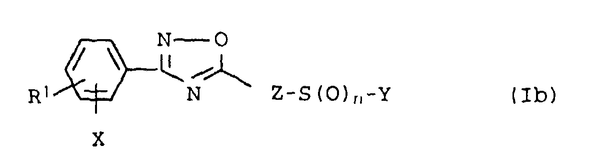 gehorchen, worin
n 0 oder 2,
X Nitro odr Amino,
Z C₁-C₄-Alkylen und
R¹ Wasserstoff, Nitro, Amino, Hydroxysulfonyl oder einen Rest der Formel S(O)ₙC₂H₄OH, worin n für 0 oder 2 steht, bedeuten und
Y die in Anspruch 1 genannte Bedeutung besitzt.

## Claims

1. A benzene derivative of the formula I 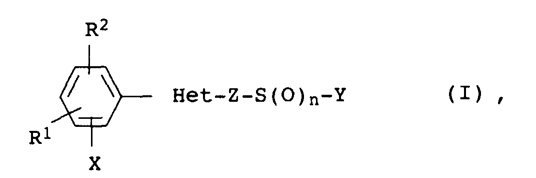 where
n is 0, 1 or 2,
X is nitro or amino,
Z is a direct bond or C₁-C₆-alkylene which can be interrupted by 1 or 2 oxygen or sulfur atoms in ether form or 1 or 2 nonadjacent imino, C₁-C₄-alkylimino or C₁-C₄-alkanoylimino groups,
Y is vinyl or a radical of the formula C₂H₄-Q, where Q is hydroxyl or a group which can be removed under alkaline reaction conditions,
R¹ and R² independently of one another are each hydrogen, C₁-C₄-alkyl, C₁-C₄-alkoxy, halogen, nitro, amino, hydroxysulfonyl or a radical of the formula Z-S(O)ₙ-Y, where n, Z and Y each have the abovementioned meanings, and
Het is a 5-membered aromatic heterocyclic radical, which can be substituted, selected from the group of heterocyclic parent structures consisting of 1,2,4-oxadiazole, 1,3,4-oxadiazole, 1,2,4-thiadiazole and 1,3,4-thiadiazole.

2. A benzene derivative as claimed in claim 1, wherein n is 0 or 2.

3. A benzene derivative as claimed in claim 1, wherein Z is C₁-C₄-alkylene which can be interrupted by an oxygen atom in ether form.

4. A benzene derivative as claimed in claim 1, wherein Y is 2-hydroxyethyl.

5. A benzene derivative as claimed in claim 1, wherein R¹ has the meanings mentioned in claim 1 and R² is hydrogen.

6. A benzene derivative as claimed in claim 1, wherein Het is 1,2,4-oxadiazole-3,5-diyl.

7. A benzene derivative as claimed in claim 1, wherein R¹ is hydrogen, nitro, amino, hydroxysulfonyl or a radical of the formula S(O)ₙC₂H₄OH, where n is 0 or 2, and R² is hydrogen.

8. A benzene derivative as claimed in claim 1, which obeys the formula Ia or Ib 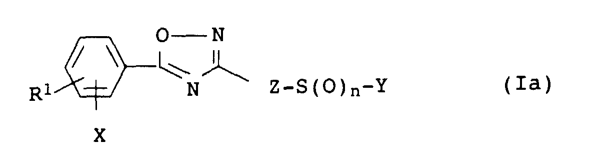 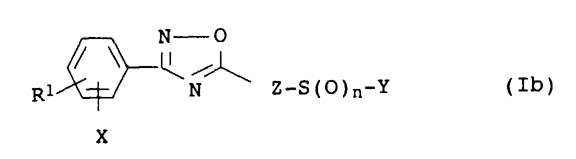 where
n is 0 or 2,
X is nitro or amino,
Z is C₁-C₄-alkylene and
R¹ is hydrogen, nitro, amino, hydroxysulfonyl or a radical of the formula S(O)ₙC₂H₄OH, where n is 0 or 2, and
Y has the meanings mentioned in claim 1.

## Revendications

1. Dérivé benzénique de formule I 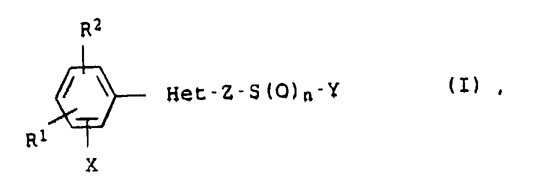 dans laquelle
n vaut 0,1 ou 2
x représente un groupement nitro ou amino
Z représente une liaison directe ou un groupement alkylène en C₁-C₆, pouvant être interrompu par 1 ou 2 atomes d'oxygène ou de soufre en fonction éther ou par 1 ou 2 groupements imino, alkylimino en C₁-C₄, ou alcanoylimino en C₁-C₄ non voisins,
Y représente un reste vinyle ou un reste de formule C₂H₄-Q, où Q est mis pour un groupement hydroxy ou pour un groupement pouvant être séparé dans des conditions réactionnelles alcalines,
R¹ et R² représentent, indépendamment l'un de l'autre, un atome d'hydrogène, un groupement alkyle en C₁-C₄, alcoxy en C₁-C₄, un atome d'halogène, un groupement nitro, amino, hydroxysulfonyle ou un reste de formule Z-S(O)ₙ-Y, où n, Z et Y prennent chacun la signification susmentionnée, et
Het représente un reste hétérocyclique aromatique à 5 maillons pouvant être substitué, choisi dans le groupe des structures de base hétérocycliques constitué du 1,2,4-oxadiazole, du 1,3,4-oxadiazole, du 1,2,4-thiadiazole et du 1,3,4-thiadiazole.

2. Dérivé benzénique selon la revendication 1, caractérisé en ce que n vaut 0 ou 2.

3. Dérivé benzénique selon la revendication 1, caractérisé en ce que Z représente un groupement alkylène en C₁-C₄, pouvant être interrompu par un atome d'oxygène en fonction éther.

4. Dérivé benzénique selon la revendication 1, caractérisé en ce que Y représente un groupement 2-hydroxyéthyle.

5. Dérivé benzénique selon la revendication 1, caractérisé en ce que R¹ prend la signification mentionnée dans la revendication 1 et R² représente un atome d'hydrogène.

6. Dérivé benzénique selon la revendication 1, caractérisé en ce que Het représente un groupement 1,2,4-oxadiazole-3,5-diyle.

7. Dérivé benzénique selon la revendication 1, caractérisé en ce que R¹ représente un atome d'hydrogène, un groupement nitro, amino, hydroxysulfonyle ou un reste de formule S(O)ₙC₂H₄OH, où n est mis pour 0 ou 2, et R² représente un atome d'hydrogène.

8. Dérivé benzénique selon la revendication 1, correspondant à la formule Ia ou Ib 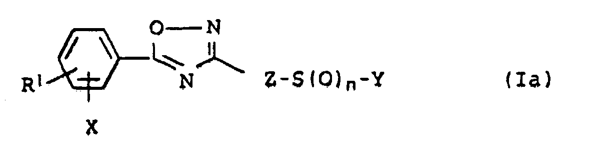 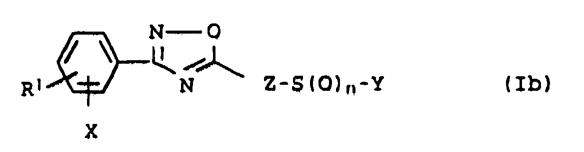 dans laquelle
n vaut 0 ou 2,
X représente un groupement amino ou nitro,
Z représente un groupement alkylène en C₁-C₄, et
R¹ représente un atome d'hydrogène, un groupement nitro, amino, hydroxysulfonyle ou un reste de formule S(O)ₙC₂H₄OH, où n est mis pour 0 ou 2 et
Y prend la signification mentionnée dans la revendication 1.
